# EUROPÄISCHE PATENTSCHRIFT

(11) **EP 0 549 640 B1**
(45) Veröffentlichungstag und Bekanntmachung des Hinweises auf die Patenterteilung: **28.12.1994**
(21) Anmeldenummer: 91916266.9
(22) Anmeldetag: 09.09.1991
(51) Int. Cl.: C07D 307/93, A61K 7/46

(54) **ISOMERE 11-OXA-TRICYCLO[7.3.0.0]DODECEN-DERIVATE, DEREN HERSTELLUNG UND VERWENDUNG ALS RIECHSTOFFE, SOWIE DIESE ENNTHALTENDE RIECHSTOFF-ZUSAMMENSETZUNGEN**
ISOMERIC 11-OXA-TRICYCLO[7.3.0.0]DODECENE DERIVATIVES, THEIR PREPARATION ANT THEIR USE AS ODORIFEROUS SUBSTANCES, AS WELL AS PERFUME COMPOSITIONS CONTAINING SUCH DERIVATIVES
DERIVES ISOMERES DE 11-OXA-TRICYCLO[7.3.0.0]DODECENE, LEUR PRODUCTION ET LEUR UTILISATION COMME MATERIERES ODORANTES, AINSI QUE COMPOSITIONS ODORANTES CONTENANT CES DERIVES

(30) Priorität: 17.09.1990 DE 4029424
(43) Veröffentlichungstag der Anmeldung: 07.07.1993
(73) Patentinhaber: Henkel Kommanditgesellschaft auf Aktien, 40191 Düsseldorf (DE)
(72) Erfinder: BRUNS, Klaus, D-4150 Krefeld-Traar (DE); DISCHMANN, Michael, D-4150 Krefeld (DE); FABER, Werner, D-4156 Willich 2 (DE); MEIERTOBERENS, Michael, D-4150 Krefeld-Fischeln (DE)
(86) Internationale Anmeldenummer: EP9101711
(87) Internationale Veröffentlichungsnummer: WO9205166

(56) Entgegenhaltungen:
- DE-A- 2 001 243
- PARFUM UND KOSMETIK, Bd. 67, Nr.11, 1986, Seiten 714-728; K. BRUNS ET AL.: 'Perfume syntheses with 2, 2, 4(2, 4, 4)-trimethylcyclopentanones. Part I.'

## Beschreibung

Die Erfindung betrifft isomere 11-Oxa-Tricyclo[7.3.0.0^{2,6}]dodecen-Derivate, ein Verfahren zu deren Herstellung sowie deren Verwendung als Riechstoffe.

11-Oxa-Tricyclo[7.3.0.0^{2,6}]dodecen-Derivate sind teilweise literaturbekannt. K.Bruns, U.Weber und M.Meiertoberens haben durch Umsetzung eines Isomerengemisches von 2,2,4(2,4,4)-Trimethyl-1-vinyl-cyclopent-1-en mit Maleinsäure- bzw. Citraconsäureanhydrid sowie Itaconsäure- und Acetylendicarbonsäuredimethylester Diels-Alder-Addukte erhalten, die nach Reduktion und Cyclisierung in tricyclische Ether mit verschiedenen holzigen Geruchsnoten überführt wurden (Parfümerie u. Kosmetik, 1986 (67) 714).

Obwohl die von Bruns et al. synthetisierten Ether gute Riechstoffeigenschaften aufweisen, besteht doch weiterhin ein Bedarf an Verbindungen mit charakteristischen qualitativ anderen Geruchsprofilen. Es wurde daher nach Verbindungen gesucht, die charakteristische neue Geruchsprofile bei gleichzeitig hoher Haftfestigkeit, Geruchsintensität und Strahlkraft aufweisen sollten.

Die Aufgabe wurde erfindungsgemäß gelöst durch neue 11-Oxa-Tricyclo[7.3.0.0^{2,6}]dodecen-Derivate. Diese wurden erhalten durch:
a) Umsetzung isomerenreiner Trimethylvinylcyclopentene (d.h. entweder 2,2,4- oder 2,4,4,-Trimethyl-1-vinyl-cyclopent-1-en) mit Maleinsäure dessen Anhydrid oder Dialkylester oder durch
b) Umsetzung isomerenreiner Trimethylvinylcyclopentene (d.h. entweder 2,2,4- oder 2,4,4,-Trimethyl-1-vinyl-cyclopent-1-en) oder Gemischen dieser Trimethylvinylcyclopentene mit Fumarsäure oder dessen Dialkylestern
zu den entsprechenden Diels-Alder-Addukten, deren Reduktion zu den entsprechenden Diolen und anschließende Cyclisierung.

Erfindungsgegenstand sind dementsprechend isomere 11-Oxa-Tricyclo[7.3.0.0^{2,6}]dodecen-Derivate der allgemeinen Formel I 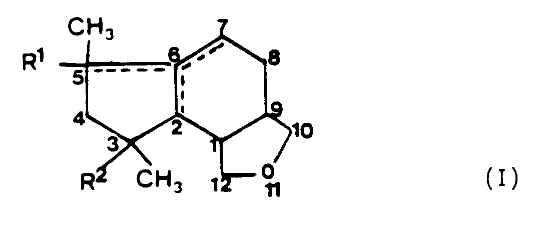
in denen:
a) R¹ eine Methylgruppe und Rest R² ein Wasserstoffatom bedeuten, der Tetrahydrofuranring bezüglich der Ringverknüpfung cis-konfiguriert ist, und in einer der Positionen C-2/C-6, C-5/C-6 oder C-6/C-7 eine C=C-Doppelbindung vorhanden ist, oder
b) R² eine Methylgruppe und Rest R¹ ein Wasserstoffatom bedeuten, der Tetrahydrofuranring bezüglich der Ringverknüpfung cis-konfiguriert ist, und in einer der Positionen C-2/C-6, C-5/C-6 oder C-6/C-7 eine C=C-Doppelbindung vorhanden ist, oder
c) einer der Reste R¹ oder R² eine Methylgruppe und der andere ein Wasserstoffatom bedeuten, der Tetrahydrofuranring bezüglich der Ringverknüpfung trans-konfiguriert ist, und in einer der Positionen C-2/C-6, C-5/C-6 oder C-6/C-7 eine C=C-Doppelbindung vorhanden ist.

Ein weiterer Gegenstand der Erfindung ist die Herstellung der isomeren 11-Oxa-Tricyclo[7.3.0.0^{2,6}]dodecen-Derivate der allgemeinen Formel I 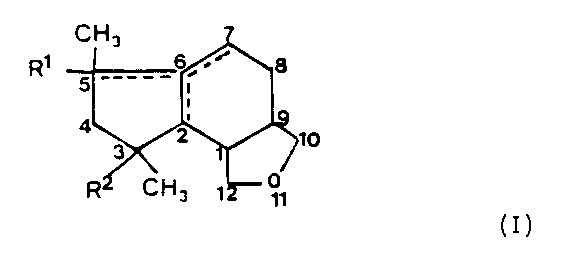
in denen:
a) R¹ eine Methylgruppe und Rest R² ein Wasserstoffatom bedeuten, der Tetrahydrofuranring bezüglich der Ringverknüpfung cis-konfiguriert ist, und in einer der Positionen C-2/C-6, C-5/C-6 oder C-6/C-7 eine C=C-Doppelbindung vorhanden ist, oder
b) R² eine Methylgruppe und Rest R¹ ein Wasserstoffatom bedeuten, der Tetrahydrofuranring bezüglich der Ringverknüpfung cis-konfiguriert ist, und in einer der Positionen C-2/C-6, C-5/C-6 oder C-6/C-7 eine C=C-Doppelbindung vorhanden ist, oder
c) einer der Reste R¹ oder R² eine Methylgruppe und der andere ein Wasserstoffatom bedeuten, der Tetrahydrofuranring bezüglich der Ringverknüpfung trans-konfiguriert ist, und in einer der Positionen C-2/C-6, C-5/C-6 oder C-6/C-7 eine C=C-Doppelbindung vorhanden ist,
durch Umsetzung eines Diens der allgemeinen Formel II, 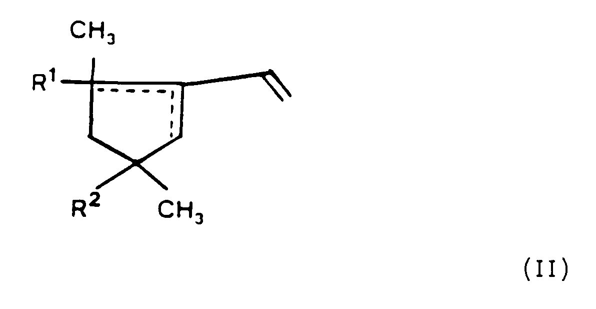
in der einer der Reste R¹ oder R² eine Methylgruppe und der andere ein Wasserstoffatom bedeuten, mit Maleinsäure, dessen Anhydrid oder einem Maleinsäuredialkylester oder mit Fumarsäure oder einem Fumarsäuredialkyl-Ester zum Diels-Alder-Addukt, Reduktion dieses Diels-Alder-Addukts zum Diol und dehydratisierende Cyclisierung des Diols zum 11-Oxa-Tricyclo[7.3.0.0^{2,6}]dodecen-Derivat.

Die Herstellung der neuen Verbindungen erfolgt nach an sich bekannten Syntheseverfahren der organischen Chemie. Als Ausgangsmaterial dient bei den über die Fumarsäureaddukte hergestellten 11-Oxa-Tricyclo[7.3.0.0^{2,6}]dodecen-Derivaten z.B. ein Isomerengemisch von 2,2,4(2,4,4)-Trimethyll-vinylcyclopent-1-en, wie es in der DE-OS 29 25 622 sowie der genannten Arbeit von Bruns et al. beschrieben ist; die Verwendung der isomerenreinen Trimethylcyclopentadiene, die aus dem Isomerengemisch destillativ gewonnen werden können, ist jedoch bevorzugt. Bei den über die Maleinsäureaddukte hergestellten 11-Oxa-Tricyclo[7.3.0.0^{2,6}]dodecen-Derivaten werden dagegen ausschließlich die isomerenreinen Trimethylcyclopentadiene eingesetzt.

Die zu Beginn der Synthesesequenz erhaltenen Diels-Alder-Addukte werden durch chemische Reduktion in die entsprechenden Diole überführt. Als Reduktionsmittel kann beispielsweise Natrium-bis-(2-methoxy-ethoxy)-aluminium-dihydrid, NaAlH₂(OCH₂CH₂OCH₃)₂, eingesetzt werden, das unter der Bezeichnung Vitride^{R} kommerziell erhältlich ist (Paesel GmbH & Co, Frankfurt a.M.). Die dehydratisierende Cyclisierung der Diole mit beispielsweise p-Toluolsulfonsäure oder Kaliumhydrogenphosphat, führt schließlich zu den erfindungsgemäßen Ethern I, die bei jeweils definierter Stereoisomerie als komplexe Regioisomerengemische erhalten werden.

Die erfindungsgemäßen Verbindungen sind Riechstoffe mit unterschiedlich nuancierten intensiv-holzigen Geruchsnoten und außergewöhnlicher Haftfestigkeit. Sie unterscheiden sich von den im Stand der Technik beschriebenen tricyclischen Ethern durch charakteristische, qualitativ deutlich andere Geruchsprofile. Die über die Fumarsäure-Addukte hergestellten erfindungsgemäßen Verbindungen sind darüber hinaus von wesentlich größerer Geruchsintensität und Strahlkraft. Der Nachgeruch (Beurteilung nach 24 Stunden) der über die Fumarsäure-Addukte hergestellten erfindungsgemäßen Ether ist sehr intensiv und von der Stellung der Methylsubstituenten abhängig. Den weitaus intensivsten Nachgeruch hat dabei die Verbindung des Beispiels 1 (vergleiche dort).

Ein weiterer Gegenstand der Erfindung ist daher die Verwendung der isomeren 11-Oxa-Tricyclo[7.3.0.0^{2,6}]dodecen-Derivate als Riechstoffe.

Die erfindungsgemäßen Verbindungen können vorteilhaft als Bausteine für neue Riechstoffkompositionen eingesetzt werden. Riechstoffkompositionen enthalten z.B. natürliche, synthetische oder partial synthetische Riechstoffe, etherische Öle und Pflanzenextrakte. Die einsetzbaren Anteile der erfindungsgemäßen Verbindungen oder deren Gemischen in Riechstoffkompositionen bewegen sich von 1 bis 50 Gewichtsprozent, bezogen auf die gesamte Mischung.

Kompositionen dieser Art können sowohl zur Parfümierung kosmetischer Präparate wie Cremes, Lotionen, Duftwässer, Aerosole, Toilettenseifen als auch in der alkoholischen Parfümerie verwendet werden. Ebenso besteht eine Einsatzmöglichkeit zur Parfümierung technischer Produkte wie Wasch- und Reinigungsmittel, Weichspüler und Textilbehandlungsmittel. Zur Parfümierung der verschiedenen Produkte werden diesen die Kompositionen in Konzentration von 0,05 bis 2 Gewichtsprozent, bezogen auf das gesamte Produkt, zugesetzt.

Die folgenden Beispiele sollen den Gegenstand der Erfinung erläutern, ohne ihn hierauf zu beschränken.

### Beispiele

1. Herstellung der Vorstufen
   Die Verbindungen der allgemeinen Formel I wurden in einer dreistufigen Synthese ausgehend vom Dien II hergestellt. Dien II lag dabei als Gemisch aus 2,2,4-Trimethyl-1-vinylcyclopent-1-en und 2,4,4-Trimethyl-1-vinylcyclopent-1-en, die aus einem 50 : 50 Gemisch von 2,2,4- und 2,4,4-Trimethylcyclopentan-1-on nach Literaturangaben (siehe oben) hergestellt wurden, vor. Daraus ergibt sich, daß sowohl die daraus hergestellten Diels-Alder-Addukte - im folgenden mit III bezeichnet - als auch die daraus erhältlichen Diole - im folgenden mit IV bezeichnet - als Isomerengemische vorliegen. In der Nomenklatur der entsprechenden Verbindungen wurde die Stellungsisomerie der drei fraglichen Methylgruppen dadurch kenntlich gemacht, daß hinter der Angabe der Stellung der drei Methylgruppen des einen Isomeren in Klammern die Stellung der Methylgruppen des anderen Isomeren aufgeführt ist.
   1.1 Herstellung der Diels-Alder-Addukte III
      1.1.1 Allgemeine Arbeitsvorschrift
         Dien II und Dienophil wurden im Molverhältnis 1 : 0,75 bis 1 : 1,2 direkt oder in einem Lösungsmittel unter Rühren 2-18 Stunden in N₂-Atmosphäre auf 80 - 125 °C erhitzt. Nach Abkühlen wurde das nicht umgesetzte Edukt und das Lösungsmittel im Vakuum abdestilliert und das verbleibende Rohprodukt gegebenenfalls im Feinvakuum destilliert. Alternativ wurde die Rohproduktlösung direkt in die Reduktionsstufe eingesetzt.
         Nach dieser allgemeinen Vorschrift wurden die im folgenden beschriebenen Verbindungen IIIa und IIIb hergestellt.
      1.1.2 Einzelsynthesen
         IIIa: (1α,9α)-11-Oxa-3,3,5(3,5,5)-trimethyl-tricyclo[7.3.0.0^{2,6}]dodec-6-en-10,12-dion
            Zu 73,5 g (0,75 mol) Maleinsäureanhydrid in 75 ml Toluol wurden bei 100 °C innerhalb 1 Stunde 112,2 g (0,83 mol) des Diens II in 110 ml Toluol getropft und 3 Stunden unter Rückfluß gerührt. Die Rohproduktlösung wurde ohne weitere Aufarbeitung in die Reduktionsstufe eingesetzt. Analytische Daten (Rohprodukt nach Entfernen des Lösungsmittels):
            - IR (Film):: 1855, 1780 cm-¹ (V_{C=O} Anhydrid)
            - ¹H-NMR (CDCl₃):: 5,3.-5,7 ppm (m, 1H, olefin. Proton)
            - MG (GC/MS):: C₁₄H₁₈O₃ ber.: 234,20 gef.: 234 (Isomere)
         IIIb: (2α,3β)-7,7,9(7,9,9)-Trimethyl-bicyclo[4.3.0]non-5-en-2,3-dicarbonsäure-di-n-butylester
            Zu 171 g (0,75 mol) Fumarsäure-di-n-butylester wurden bei 120 °C innerhalb 1 Stunde 122,4 g (0,9 mol) des Diens II getropft und weitere 6 Stunden bei 120 °C gerührt. Das Rohprodukt wurde ohne weitere Aufarbeitung in die Reduktionsstufe eingesetzt.
            Analytische Daten:
            - IR (Film):: 1735 cm⁻¹ (V_{C=O} Ester)
            - MG (GC/MS):: C₂₂H₃₆O₄ ber.: 364,53 gef.: 364 (Isomere)
   1.2 Herstellung der Diole IV
      1.2.1 Allgemeine Arbeitsvorschrift
         Die Diels-Alder-Addukte III wurden in toluolischer Lösung unter N₂-Atmosphäre während ca. 1 Stunde mit einer ca. 35 %igen Vitride^{R}-Lösung in Toluol versetzt oder zu einer 70 %igen Vitride^{R}-Lösung getropft. Die Reaktionen verliefen exotherm bei 45-60 °C. Anschließend wurde für weitere 2 bis 4 Stunden unter Rückfluß gerührt.
         Aufarbeitung: Die abgekühlte Reaktionslösung wurde mit 5 ml Aceton und danach bis zu Ausflockung des Aluminat-Niederschlags mit ca. 180 - 210 ml destilliertem Wasser versetzt. Die verbleibende toluolische Lösung wurde mit 3n-Salzsäure, gesättigter NaHCO₃- und gesättigter NaCl-Lösung gewaschen, über Natriumsulfat getrocknet und das Toluol im Vakuum entfernt.
         Nach dieser allgemeinen Vorschrift wurden die im folgenden beschriebenen Verbindungen IVa und IVb hergestellt.
      1.2.2 Einzelsynthesen
         IVa: (2α,3α:)-2,3-Di-(hydroxymethyl)-7,7,9(7,9,9)-trimethyl-bicyclo[4.3.0]non-5-en
            Die Rohproduktlösung IIIa wurde während 1 Stunde mit einer Lösung aus 470 ml (1,68 mol) Vitride^{R} (70 %ig in Toluol) in 500 ml Toluol versetzt und anschließend 2 Stunden unter Rückfluß gerührt. Aufarbeitung wie oben beschrieben; Rohprodukt: 138,9 g.
            Analytische Daten:
            - IR (Film): 3310, 1030 cm⁻¹ (v_{O-H}, v_{C-O} Alkohol)
            - 1H-NMR (CDCl₃):: 3,2-3,8 ppm (m, 6H, -CH₂-OH) 5,1-5,35 ppm (m, 1H, olefin. Proton)
            - MG (GC/MS):: C₁₄H₂₄O₂ ber.: 224,35 gef.: 224 (Isomere)
         IVb: (2α,3β)-2,3-Di-(hydroxymethyl)-7,7,9(7,9,9)-trimethyl-bicyclo[4.3.0]non-5-en
            Das Rohprodukt IIIb gelöst in 500 ml Toluol wurde während 1 Stunde mit einer Lösung aus 470 ml (1,68 mol) Vitride^{R} (70 %ig in Toluol) in 500 ml Toluol versetzt und anschließend 2 Stunden unter Rückfluß gerührt.
            Aufarbeitung wie oben beschrieben; Rohprodukt: 137,5 g.
            Analytische Daten:
            - IR (Film):: 3330, 1040 cm-¹ (V_{O-H}, v_{C-O} Alkohol)
            - MG (GC/MS):: C₁₄H₂₄O₂ ber.: 224,35 gef.: 224 (Isomere)
            Die Produkte IVa und IVb konnten ohne weitere Aufarbeitung in der nachfolgenden Cyclisierungsstufe eingesetzt werden.
2. Herstellung der erfindungsgemäßen Verbindungen I
   Die erfindungsgemäßen Ether I wurden aus dem Diolen IV durch dehydratisierende Cyclisierung hergestellt.
   2.1. Allgemeine Arbeitsvorschrift
      Das auf 55 °C erwärmte rohe Diol IV wurde bei einer Badtemperatur von 180 °C und einem Druck von 1 mbar zu ca. 0,14 Moläquivalenten KHSO₄ getropft. Das gebildete Cyclisierungsprodukt I wurde dabei gleichzeitig über Kopf abdestilliert, wobei sich eine Brüdentemperatur von 95 - 105 °C einstellte. Nach beendeter Zugabe wurden weitere 1,5 Stunden bei 190 °C und 1 mbar destilliert und das Rohprodukt anschließend im Feinvakuum fraktioniert.
      Nach dieser allgemeinen Vorschrift wurden die im folgenden beschriebenen Verbindungen des Vergleichsbeispiels 1 sowie der Beispiele 1 bis 5 hergestellt. Unter den Bedingungen der Cyclisierung trat gleichzeitig eine teilweise Isomerisierung der C=C-Doppelbindung ein; dies geht auch aus der jeweiligen Bezeichnung der Verbindungen Ia und Ib hervor.
   2.2 Einzelsynthesen
      Vergleichsbeispiel 1
         Herstellung eines Isomerengemisches (Ia) bestehend aus: (1α, 9α)-11-Oxa-3,3,5(3,5,5)-trimethyl-tricyclo[7.3.0.0^{2,6}]dodec-6-en (A), (1α,9α)-11-Oxa-3,3,5(3,5,5)-trimethyl-tricyclo[7.3.0.0^{2,6}] ^{2,6}-dodecen (B) und (1α,9α)-11-Oxa-3,3,5-trimethyl-tricyclo[7.3.0.0^{2,6}]dodec-5-en (C)
         Reaktion: 138,9 g (ca. 0,62 mol) IVa, 12 g (0,088 mol) KHSO₄, Zutropfzeit 5 Stunden.
         Kp_{0,25}: 62-70 °C
         Ausbeute: 92,6 g = 60 % der Theorie bezogen auf Maleinsäureanhydrid
         Zusammensetzung: A : B : C = 1:3:3
         Geruch: holzig, erdig, camphrig, Ceder-, Dynamon-, Birkenteer-Note
         Analytische Daten:
         - IR (Film):: 1080, 912 cm⁻¹ (V_{C-O-C(as), Ring} cycl. Ether
         - 1H-NMR (CDCl₃):: 1,67 ppm ("s", 3H, -C=C-CH₃) 3,0-4,0 ppm (m, 4H, -CH₂-O-CH₂) 5,3-5,45 ppm (m, 1H, olefin. Proton)
         - MG (GC/MS):: C₁₄H₂₂O ber.: 206,33 gef.: 206 (Isomere)
      Beispiel 1
         Herstellung eines Isomerengemisches (Ib) bestehend aus (1α,9β)-11-Oxa-3,3,5(3,5,5)-trimethyl-tricyclo[7.3.0.0^{2,6}]dodec-6-en (A) und (1α,9β)-11-Oxa-3,3,5(3,5,5)-trimethyl-tricyclo[7.3.0.0^{2,6}] ^{2,6}dodecen (B)
         Reaktion: 137,5 g (ca. 0,61 mol) IVb, 12 g (0,088 mol) KHSO₄, Zutropfzeit: 5 Stunden
         Kp_{0,3}: 75 - 78 °C
         Ausbeute: 62,7 g = 40 % der Theorie bezogen auf Fumarsäure-di-n-butylester
         Zusammensetzung: A : B = 2 : 1
         - IR (Film):: 1020, 900 cm-¹ (V_{C-O-Cas}, Ring cycl. Ether)
         - ¹H-NMR (CDCl₃):: 3,2 - 4,2 ppm (m, 4H, -CH₂-O-CH₂-) 5,3 - 5,4 ppm (m, 1H, olefin. Proton)
         - MG (GC/MS):: C₁₄H₂₂O ber.: 206,33 gef.: 206 (Isomere)
         Geruch: holzig, narkotisch, starke Jonon-, Boisambrene-Note; doppelt so stark wie die analoge cis-Verbindung aus Vergleichsbeispiel 1.
         Durch Einsetzen von isomerenreinem 2,2,4-Trimethyl-1-vinylcyclopent-1-en bzw. 2,4,4-Trimethyl-1-vinylcyclopent-1-en zu Beginn der Synthesesequenz II -> III -> IV wurden die isomerenreinen Verbindungen des Typs IVa und IVb erhalten. Diese Verbindungen wurden ebenfalls nach der oben beschriebenen allgemeinen Vorschrift cyclisiert (Beispiele 2 bis 5).
      Beispiel 2
         Herstellung eines Isomerengemisches (Ia) bestehend aus: (1α,9α)-11-Oxa-3,5,5-trimethyl-tricyclo[7.3.0.0^{2,6}]dodec-6-en-(A) und (1α,9α)--11-Oxa-3,5,5-trimethyl-tricyclo[7.3.0.0.^{2,6}] ^{2,6}dodecen (B)
         Zusammensetzung: A : B = 1 : 2
         Geruch: holzig, erdig, Dynamon-, Ceder-Note
         Analytische Daten:
         - IR (Film):: entsprechend Vergleichsbeispiel 1
         - ¹H-NMR (CDCl₃):: 3,2-4,0 ppm (m, 4H, -CH₂-O-CH₂-) 5,3-5,45 ppm (m, 1H, olefin. Proton)
         - MG (GC/MS):: entsprechend Vergleichsbeispiel 1
      Beispiel 3
         Herstellung eines Isomerengemisches (Ib) bestehend aus: (1α,9β)-11-Oxa-3,5,5-trimethyl-tricyclo[7.3.0.0^{2,6}]dodec-6-en (A) und (1α,9β)-11-Oxa-3,5,5-trimethyl-tricyclo[7.3.0.0.^{2,6}] ^{2,6}dodecen (B)
         Zusammensetzung: A : B = 1 : 1,5
         Geruch: holzig, narkotisch, starke Jonon-, Boisambrene-Note, leicht fruchtig; sehr ähnlich wie in Beispiel 1, jedoch noch größere Strahlkraft
         Analytische Daten:
         - IR (Film): entsprechend Beispiel 1
         - ¹H-NMR (CDCl₃):: 3,2 - 4,2 ppm (m, 4H,-CH₂-O-CH₂-) 5,3 - 5,4 ppm (m, 1H, olefin. Proton)
         - MG (GC/MS):: entsprechend Beispiel 1
      Beispiel 4
         Herstellung eines Isomergemisches (Ia) bestehend aus: (1α,9α)-11-Oxa-3,3,5-trimethyl-tricyclo[7.3.0.0^{2,6}]dodec-6-en (A), (1α,9α)-11-Oxa-3,3,5-trimethyl-tricyclo[7.3.0.0^{2,6}] ^{2,6}dodecen (B) und (1α,9α)-11-Oxa-3,3,5-trimethyl-tricyclo[7.3.0.0^{2,6}]dodec-5-en (C)
         Zusammensetzung: A : B : C = 1 : 4 : 7
         Geruch: fruchtig, holzig, narkotisch, camphrig, deutlich stärker als in Beispiel 2
         Analytische Daten:
         - IR (Film):: 1080, 912 cm⁻¹ (V_{C-O-C(as), Ring} cycl. Ether
         - 1H-NMR (CDCl₃):: 1,68 ppm ("s", 3H, -C=C-CH₃) 3,0-4,1 ppm (m, 4H, -CH₂-O-CH₂) 5,4 ppm (m, 1H, olefin. Proton)
         - MG (GC/MS):: C₁₄H₂₂O ber.: 206,33 gef.: 206 (Isomere)
      Beispiel 5
         Herstellung eines Isomerengemisches bestehend aus: (1α,9β)-11-Oxa-3,3,5-trimethyl-tricyclo[7.3.0.0^{2,6}]dodec-6-en (A) und (1α,9β)-11-Oxa-3,3,5-trimethyl-tricyclo[7.3.0.0^{2,6}] ^{2,6}dodecen (B)
         Zusammensetzung: A : B = 1 : 1
         Geruch: Teer-, Schiffsplanken-, verbranntes Holz-Note, technisch
         - IR (Film):: entsprechend Beispiel 1
         - ¹H-NMR (CDCl₃):: 3,2 - 4,1 ppm (m, 4H, -CH₂-O-CH₂-) 5,3 - 5,45 ppm (m, 1H, olefin. Proton)
         - MG (GC/MS):: entsprechend Beispiel 1

### Kompositionsbeispiel

### Herren-Note Typ "Polo"

| | Gew.-Teile |
|---|---|
| Citronenöl 67 | 200 |
| Lavendelöl echt | 30 |
| Eucalyptusöl Globulus | 40 |
| Wacholderbeeröl | 10 |
| Muskatnußöl | 25 |
| Aldehyd C-12 (MNA) | 2 |
| Phenylacetaldehyd-dimethylacetal | 3 |
| Methyl-dihydrojasmonat | 170 |
| Jasmonan (Henkel) | 10 |
| o-tert.Butyl-cyclohexylacetat | 3 |
| Cyclovertal (Henkel) | 2 |
| Cumarin | 25 |
| Lyral | 60 |
| α-n-Methyljonon | 20 |
| Iso-E-Super (IFF) | 100 |
| Kephalis (Roure Bertrand) | 30 |
| Muskateller Salbeiöl | 20 |
| Cassis synth. (Firmenich) | 10 |
| Ambroxan 10 %ig (Henkel) | 15 |
| Eugenol | 15 |
| Moschus Keton | 30 |
| Galaxolide 50 (IFF) | 70 |
| Cyclohexylsalicylat (Henkel) | 10 |
| Evernyl (Roure Bertrand) | 20 |
| Ib-1 (Beispiel 1) oder Ia-1 (Beispiel 4) | 70 |
| | 1̅0̅0̅0̅ |

## Patentansprüche

1. Isomere 11-Oxa-Tricyclo[7.3.0.0^{2,6}]dodecen-Derivate der allgemeinen Formel I 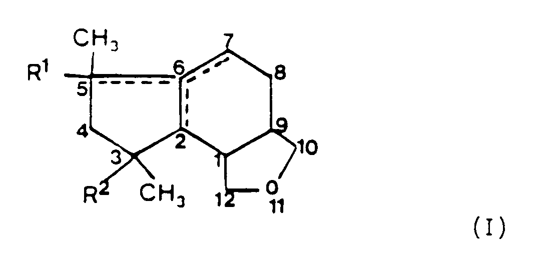 in denen:
a) R¹ eine Methylgruppe und Rest R² ein Wasserstoffatom bedeuten, der Tetrahydrofuranring bezüglich der Ringverknüpfung cis-konfiguriert ist, und in einer der Positionen C-2/C-6, C-5/C-6 oder C-6/C-7 eine C=C-Doppelbindung vorhanden ist, oder
b) R² eine Methylgruppe und Rest R¹ ein Wasserstoffatom bedeuten, der Tetrahydrofuranring bezüglich der Ringverknüpfung cis-konfiguriert ist, und in einer der Positionen C-2/C-6, C-5/C-6 oder C-6/C-7 eine C=C-Doppelbindung vorhanden ist, oder
c) einer der Reste R¹ oder R² eine Methylgruppe und der andere ein Wasserstoffatom bedeuten, der Tetrahydrofuranring bezüglich der Ringverknüpfung trans-konfiguriert ist, und in einer der Positionen C-2/C-6, C-5/C-6 oder C-6/C-7 eine C=C-Doppelbindung vorhanden ist.

2. Verfahren zur Herstellung isomerer 11-Oxa-Tricyclo[7.3.0.0^{2,6}]dodecen-Derivate der allgemeinen Formel I 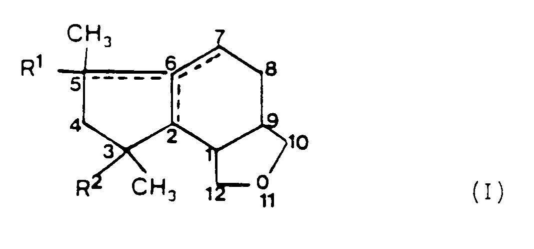 in denen:
a) R¹ eine Methylgruppe und Rest R² ein Wasserstoffatom bedeuten, der Tetrahydrofuranring bezüglich der Ringverknüpfung cis-konfiguriert ist, und in einer der Positionen C-2/C-6, C-5/C-6 oder C-6/C-7 eine C=C-Doppelbindung vorhanden ist, oder
b) R² eine Methylgruppe und Rest R¹ ein Wasserstoffatom bedeuten, der Tetrahydrofuranring bezüglich der Ringverknüpfung cis-konfiguriert ist, und in einer der Positionen C-2/C-6, C-5/C-6 oder C-6/C-7 eine C=C-Doppelbindung vorhanden ist, oder
c) einer der Reste R¹ oder R² eine Methylgruppe und der andere ein Wasserstoffatom bedeuten, der Tetrahydrofuranring bezüglich der Ringverknüpfung trans-konfiguriert ist, und in einer der Positionen C-2/C-6, C-5/C-6 oder C-6/C-7 eine C=C-Doppelbindung vorhanden ist.
durch Umsetzung eines Diens der allgemeinen Formel II, 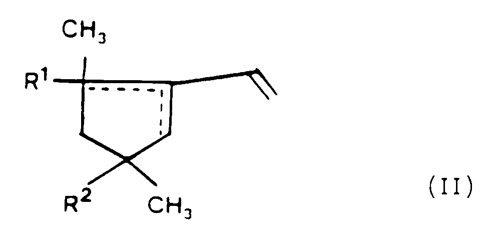 in der einer der Reste R¹ oder R² eine Methylgruppe und der andere ein Wasserstoffatom bedeuten, mit Maleinsäure, dessen Anhydrid oder einem Maleinsäuredialkylester oder mit Fumarsäure oder einem Fumarsäuredialkyl-Ester zum Diels-Alder-Addukt, Reduktion dieses Diels-Alder-Addukts zum Diol und dehydratisierende Cyclisierung des Diols zu einem 11-Oxa-Tricyclo[7.3.0.0^{2,6}]dodecen-Derivat gemäß Anspruch 1.

3. Riechstoffkompositionen, gekennzeichnet durch einen Gehalt an einem 11-Oxa-Tricyclo[7.3.0.0^{2,6}]dodecen-Derivat des Anspruchs 1 oder einem Gemisch dieser isomeren 11-Oxa-Tricyclo[7.3.0.0^{2,6}]dodecen-Derivate in einer Menge von 1 bis 50 Gewichtsprozent, bezogen auf die gesamte Komposition.

4. Verwendung der isomeren 11-Oxa-Tricyclo[7.3.0.0^{2,6}]dodecen-Derivate des Anspruchs 1 als Riechstoffe, z.B. in kosmetischen Präparaten, technischen Produkten oder der alkoholisclen Parfümerie.

5. Verwendung von Riechstoffkompositionen gemäß Anspruch 3 zur Parfümierung kosmetischer Präparate, technischer Produkte oder in der alkoholischen Parfümerie.

## Claims

1. Isomeric 11-oxatricyclo[7.3.0.0^{2,6}]dodecene derivatives corresponding to general formula I 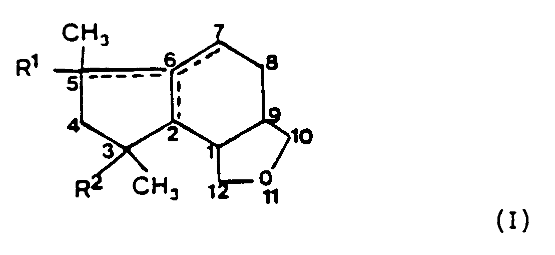 in which
a) R¹ is a methyl group and R² is a hydrogen atom, the tetrahydrofuran ring is cis-configured in its ring linkage and a C=C double bond is present in one of the positions C-2/C-6, C-5/C-6 or C-6/C-7
or
b) R² is a methyl group and R¹ is a hydrogen atom, the tetrahydrofuran ring is cis-configured in its ring linkage and a C=C double bond is present in one of the positions C-2/C-6, C-5/C-6 or C-6/C-7
or
c) one of the substituents R¹ or R² is a methyl group and the other is a hydrogen atom, the tetrahydrofuran ring is trans-configured in its ring linkage and a C=C double bond is present in one of the positions C-2/C-6, C-5/C-6 or C-6/C-7.

2. A process for the production of isomeric 11-oxatricyclo[7.3.0.0^{2,6}]dodecene derivatives corresponding to general formula I 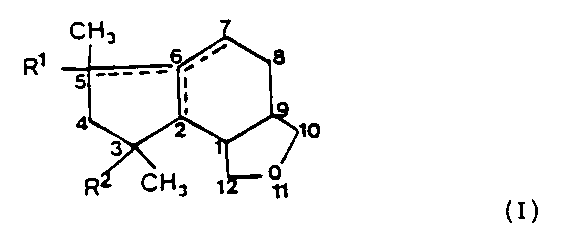 in which
a) R¹ is a methyl group and R² is a hydrogen atom, the tetrahydrofuran ring is cis-configured in its ring linkage and a C=C double bond is present in one of the positions C-2/C-6, C-5/C-6 or C-6/C-7
or
b) R² is a methyl group and R¹ is a hydrogen atom, the tetrahydrofuran ring is cis-configured in its ring linkage and a C=C double bond is present in one of the positions C-2/C-6, C-5/C-6 or C-6/C-7
or
c) one of the substituents R¹ or R² is a methyl group and the other is a hydrogen atom, the tetrahydrofuran ring is trans-configured in its ring linkage and a C=C double bond is present in one of the positions C-2/C-6, C-5/C-6 or C-6/C-7,
by reaction of a diene corresponding to general formula II 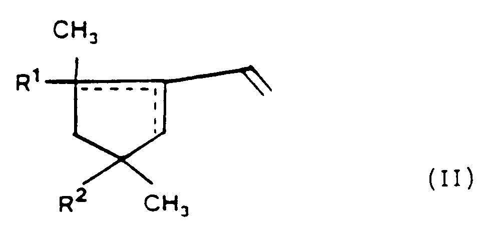 in which one of the substituents R¹ or R² is a methyl group and the other is a hydrogen atom,
with maleic acid, maleic anhydride or a maleic acid dialkyl ester or with fumaric acid or a fumaric acid dialkyl ester to form the Diels-Alder adduct, reduction of the Dieis-Alder adduct to the diol and dehydrating cyclization of the diol to form an 11-oxatricyclo[7.3.0.0^{2,6}]dodecene derivative according to claim 1.

3. Fragrance compositions, characterized by a content of an 11-oxatricyclo[7.3.0.0^{2,6}]dodecene derivative according to claim 1 or a mixture of these isomeric 11-oxatricyclo[7.3.0.0^{2,6}]dodecene derivatives in a quantity of 1 to 50% by weight, based on the composition as a whole.

4. The use of the isomeric 11-oxatricyclo[7.3.0.0^{2,6}]dodecene derivatives according to claim 1 as fragrances, for example in cosmetic preparations, technical products or alcohol-based perfumery.

5. The use of the fragrance compositions claimed in claim 3 for perfuming cosmetic preparations and technical products or in alcohol-based perfumery.

## Revendications

1. Dérivés de 11-oxa-tricyclo[7.3.0.0^{2,6}]dodécène isomères de formule générale (I) 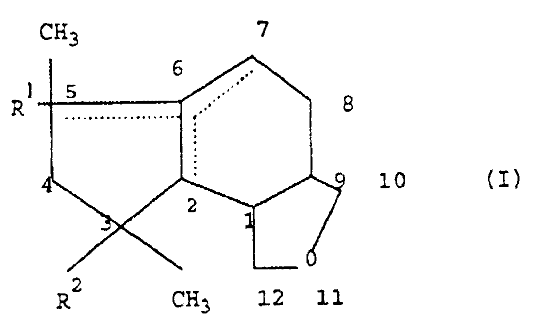 dans laquelle :
a) R¹ signifie un méthyle et le reste R² un hydrogène, le cycle tétrahydrofuranne étant en configuration cis par rapport à l'accrochage du cycle et une double liaison C=C est présente en une des positions C₂/C₆, C₅/C₆ ou C₆/C₇ ou
b) R² signifie un méthyle et le reste R¹ un atome d'hydrogène, le cycle tétrahydrofuranne est en configuration cis par rapport à l'accrochage du cycle et une double liaison C=C est présente en une des positions C₂/C₆, C₅/C₆ ou C₆/C₇
c) un des restes R¹ ou R² signifie un méthyle et l'autre un atome d'hydrogène, le cycle tétrahydrofuranne est en configuration trans par rapport à l'accrochage du cycle et en une des positions C₂/C₆, C₅/C₆ ou C₆/C₇, une double liaison C=C est présente.

2. Procédé d'obtention de dérivés du 11-oxa-tricyclo[7.3.0.0^{2,6}]dodécène isomères de formule générale (I) : 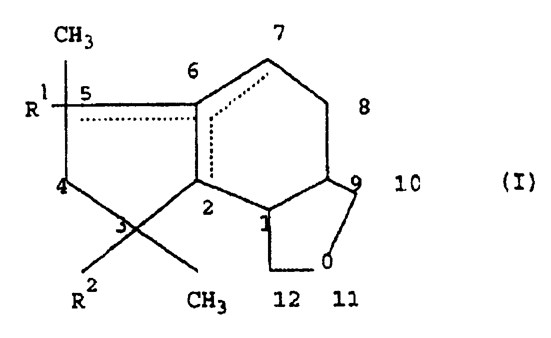 dans laquelle :
a) R¹ signifie un méthyle et le reste R² un hydrogène, le cycle tétrahydrofuranne étant en configuration cis par rapport à l'accrochage du cycle et une double liaison C=C est présente en une des positions C₂/C₆, C₅/C₆ ou C₆/C₇,
b) R² signifie un méthyle et le reste R¹ est un atome d'hydrogène, le cycle tétrahydrofuranne est en configuration cis par rapport à l'accrochage du cycle et en une des positions C₂/C₆, C₅/C₆ ou C₆/C₇ une double liaison C=C est présente, ou
c) un des restes R¹ ou R² signifie un méthyle et l'autre un atome d'hydrogène, le cycle tétrahydrofuranne est en configuration trans par rapport à l'accrochage du cycle et en une des positions C₂/C₆, C₅/C₆ ou C₆/C₇ une double liaison C=C est présente.
par mise en réaction d'un diène de formule générale II : 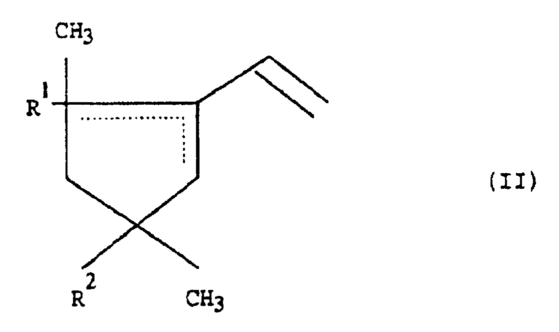 dans laquelle un des restes R¹ ou R² signifie un méthyle et l'autre un atome d'hydrogène
avec de l'arthydride maléique, son anhydride ou un ester dialcoylique d'acide maléique, ou avec de l'acide fumarique ou un ester dialcoylique d'acide fumarique, en un composé d'addition selon Diels-Alder, réduction de ce composé d'addition selon Diels-Alder en diol et cyclisation déshydratante du diol en un dérivé du 11-oxa-tricyclo[7.3.0.0^{2,6}] dodécène selon la revendication 1.

3. Compositions de substances odorantes caractérisées par une teneur en dérivé de 11-oxa-tricyclo[7.3.0.0^{2,6}]dodécène de la revendication 1 ou d'un mélange de ces dérivés du 11-oxa-tricyclo [7.3.0.0^{2,6}] dodécène isomères, en quantités allant de 1 à 50 % en poids, rapporté à la composition totale.

4. utilisation des dérivés de 11-oxa-tricyclo[7.3.0.0^{2,6}]dodécène isomères de la revendication 1 comme substances odorantes par exemple dans des préparations cosmétiques, des produits techniques ou en parfumerie alcoolisée.

5. Utilisation de compositions de substances odorantes selon la revendication 3 en vue de l'aromatisation de préparations cosmétiques, de produits techniques ou en parfumerie alcoolisée.
